# EUROPEAN PATENT SPECIFICATION

(11) **EP 3 706 830 B1**
(45) Date of publication and mention of the grant of the patent: **07.08.2024**
(21) Application number: 18795865.7
(22) Date of filing: 04.10.2018
(51) Int. Cl.: A61M 5/20, A61M 5/32

(54) **DRUG DELIVERY DEVICE WITH PLACEMENT AND FLOW SENSING**
ARZNEIMITTELABGABEVORRICHTUNG MIT POSITIONIERUNGS- UND DURCHFLUSSMESSUNG
DISPOSITIF D'ADMINISTRATION DE MÉDICAMENT AVEC DÉTECTION DE POSITIONNEMENT ET DE DÉBIT

(30) Priority: 06.11.2017 US 201762582268 P
(43) Date of publication of application: 16.09.2020
(73) Proprietor: Amgen Inc., Thousand Oaks, California 91320-1799 (US)
(72) Inventor: GROSZMANN, Daniel, Eduardo, Belmont, MA 02478 (US); GIBSON, Scott, Robert, Granada Hills, CA 91344 (US)
(74) Representative: J A Kemp LLP
(86) International application number: PCT/US2018/054289
(87) International publication number: WO 2019/089178

(56) References cited:
- EP-A2- 3 000 497
- WO-A1-2015/187793
- WO-A1-2017/120178
- US-A1- 2016 235 916

## Description

### FIELD OF THE DISCLOSURE

The present disclosure relates to drug delivery devices and, more particularly, drug delivery devices with electronic control.

### BACKGROUND

Drugs can be administered through the use of drug delivery devices such as autoinjectors or on-body injectors. Autoinjectors and on-body injectors may be used to help automate the injection and delivery or administration process, thereby simplifying the process for certain patient groups or sub-groups for which use of the syringe/vial combination or pre-filled syringe systems would be disadvantageous, whether because of physiological or psychological impediments.

Even after receiving specified training, however, some patients and/or caregivers can experience challenges while using autoinjectors and/or on-body injectors. Such challenges may relate to placement of the device on the person and/or device activation. The user may be uncertain if the device is located properly prior to operation. The user may inadvertently move the device before a full dose can be dispensed or may dispense the drug at a depth that is not optimal. The user may also be uncertain whether his/her sequence of actions has correctly operated the drug delivery device. US 2016/0235916 discloses a medical injector with compliance tracking and monitoring.

### SUMMARY

As set forth in more detail below, the present disclosure sets forth a drug delivery system embodying advantageous improvements to ensure that the drug delivery device is properly placed prior to drug delivery, and to inform the patient if and when that placement has been disturbed during drug delivery. The invention is defined in the claims. Further aspects and preferred embodiments are defined in the dependent claims. Any aspects, embodiments and examples of the present disclosure which do not fall under the scope of the appended claims do not form part of the invention and are merely provided for illustrative purposes.

In some embodiments, a drug delivery device is described herein that includes a housing defining an interior cavity and a distal surface for contacting a patient during a dispensing operation of the drug delivery device. The device further includes a primary container disposed in the interior cavity of the housing and adapted to contain a drug; a cannula having an electrically conductive portion, a storage state where a terminal end thereof is inside the housing, and a delivery state where the terminal end thereof extends out through an opening in the distal surface of the housing. The device further includes a fluid flow path for the drug including the primary container and the cannula; a delivery mechanism disposed in the housing for selectively forcing the drug through the fluid flow path out of the primary container and through the cannula for delivery to a patient when the cannula occupies the delivery state; a flow measurement device configured to measure a characteristic of the flow of the drug forced through the fluid flow path; and an electrode disposed on the distal surface of the housing. Further the device includes a controller carried by the housing and electrically connected to the flow measurement device, the cannula, and the electrode. The controller is configured to determine whether the drug was successfully delivered to a patient by: determining that an electrical circuit including the cannula and the electrode has closed upon application of the electrode on the distal surface of the housing onto a patient's skin and insertion of the cannula into the patient; and determining, based on the measurements of the flow measurement device and in combination with recognizing the closed electrical circuit, whether a full dose of the drug has been forced through the fluid flow path and delivered to the patient.

In further embodiments, the flow measurement device can be a flow sensor disposed within the fluid flow path to measure the flow of the drug therethrough.

In further embodiments, the electrically conductive portion of the cannula can be at a distal end thereof, and the controller is configured to determine a depth of the distal end of the cannula in the delivery state based on readings of the electrical circuit.

In further embodiments, the drug delivery device can further include a communication module and the controller can be configured to send a confirmation signal with the communication module in response to determining that the electrical circuit was closed while a full dose of the drug was dispensed.

In further embodiments, the drug delivery device can further include a communication module and the controller can be configured to send a fault signal in response to determining that less than a full dose of the drug was dispensed or that the electrical circuit opened while the drug was being dispensed.

### BRIEF DESCRIPTION OF THE DRAWINGS

The above needs are at least partially met through provision of the drug delivery device embodiments described in the following detailed description, particularly when studied in conjunction with the drawings, wherein:
Fig. 1 is a perspective view of an embodiment of an on-body drug delivery device according to the present disclosure;
Fig. 2 is a cross-sectional side view of the drug delivery device of Fig. 1;
Fig. 3 is a bottom plan view of the drug delivery device of Figs. 1 and 2;
Fig. 4 is a perspective view an embodiment of an autoinjector drug delivery device according to the present disclosure;
Fig. 5 is a cross-sectional side view of the drug delivery device of Fig. 4;
Fig. 6 is a block diagram of a controller of the drug delivery device of Figs. 4 and 5; and
Fig. 7 is an end view of the drug delivery device of Figs. 4 - 6.

Skilled artisans will appreciate that elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present invention. Also, common but well-understood elements that are useful or necessary in a commercially feasible embodiment are often not depicted in order to facilitate a less obstructed view of these various embodiments. It will further be appreciated that certain actions and/or steps may be described or depicted in a particular order of occurrence while those skilled in the art will understand that such specificity with respect to sequence is not actually required. It will also be understood that the terms and expressions used herein have the ordinary technical meaning as is accorded to such terms and expressions by persons skilled in the technical field as set forth above except where different specific meanings have otherwise been set forth herein

### DETAILED DESCRIPTION

The present application is directed to a simple and effective drug delivery device and method of using the device, wherein the device includes an on-board controller to determine when proper placement on the patient's body has occurred as well as an amount of drug delivered to the patient. The controller can also be used to determine if and when the proper placement has been disturbed during drug delivery and/or if less than a full dose of the drug has been dispensed.

The drug delivery devices described herein achieve these functionalities by providing an electrical circuit that is completed (e.g., closed) only upon proper placement of the device on the patient's skin and proper operation of the device to subcutaneously position the cannula within the patient. The electrical circuit includes two contacts, each including an electrode, that engage the patient's body in order to close the circuit. If the electrodes maintain contact with the patient's skin throughout a drug delivery operation, the device can confirm proper positioning.

One of the contacts includes a drug delivery cannula, which is configured to penetrate the patient's skin for subcutaneous drug delivery. The other contact is an electrode mounted on a surface of the drug delivery device, or a component attached thereto, that also contacts the patient during drug delivery. In this case, the surface carrying the second electrode can include a distal surface of the device through which the drug delivery cannula extends. With this configuration, when the drug delivery cannula penetrates the patient's skin and the second electrode simultaneously contacts the patient, the electrical circuit is closed. The controller can monitor the electrical circuit to determine when it is closed to thereby indicate that the drug delivery device has been properly placed. Thereafter, a user can operate the drug delivery device, or the device can be configured to autonomously operate, to deliver the drug to the patient. Further, during delivery, the controller can monitor the electrical circuit to determine, or be configured to be alerted, if and when the electrical circuit opens or breaks. Based on this determination, the controller may stop drug delivery, alert the user with a notification device, transmit an electronic message to a remote device, etc. Thus, not only does the disclosed technology ensure proper placement for drug delivery but it can also advantageously give the user a sense of comfort that proper placement is maintained throughout the delivery process.

The drug delivery devices described herein achieve further functionalities by monitoring the flow of drug during a delivery operation. The drug delivery devices may include a flow measurement device that couples to a fluid flow path and/or delivery mechanism in the drug delivery device. The flow measurement device can be in communication with the controller so that the controller can determine whether a full dose of the drug was delivered during a delivery operation. The flow measurement device can take any suitable form.

Used in combination, drug delivery devices can be utilized to confirm proper placement and full dose delivery. Moreover, the disclosed technology can be leveraged to monitor, track, and manage compliance

The present disclosure is related to drug delivery devices in general and provides two exemplary embodiments including an on-body injector and an autoinjector. The technology described herein, however, can also be incorporated into other drug delivery devices, such as pumps, patches, and so forth.

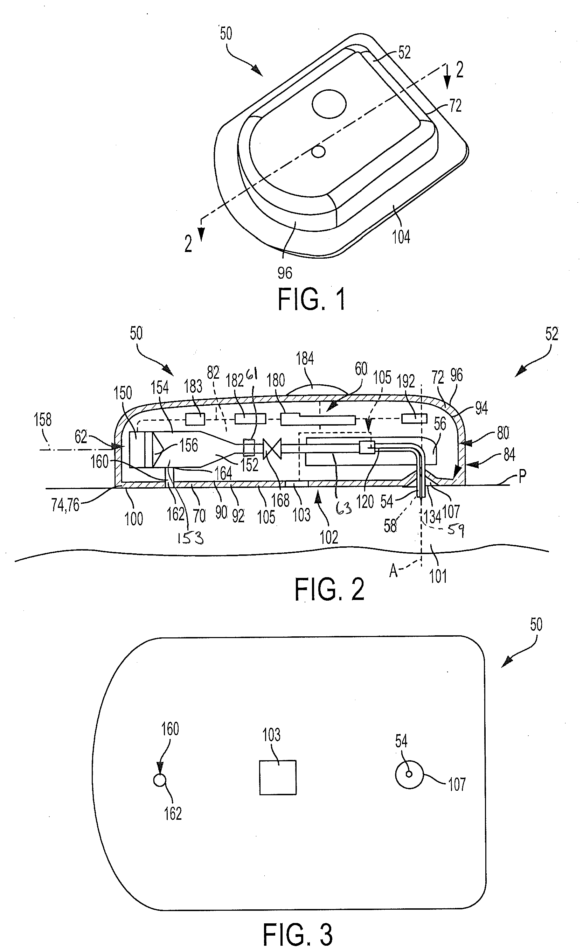

Figs. 1-3 illustrate one version of a wearable drug delivery device 50 (e.g., an on-body injector) constructed in accordance with the principles of the present disclosure. As shown in Fig. 1, the device 50 includes a housing 52 having a dome portion 72 with an exterior surface 96 that is attachable to a patient 101 (Fig. 2) with adhesive, for example. If desired, the housing 52 can include an adhesive 104 applied thereto for securing the device 50 to a patient. Alternatively, the adhesive 104 can be disposed on a patch coupled to the device 50. In such a configuration, the patch can include openings corresponding to the components described below.

Turning now to Fig. 2, in addition to the dome 72, the device housing 52 includes a plate portion 70 that is applied against a patient's skin. The dome 72 that is attached to the plate 70, preferably by a seal at an interface between a peripheral edge 74 of the plate 70 and a peripheral edge 76 of the dome 72.

The housing 52 has an interior surface 80 defining a sealed space 82 and an exterior surface 84. In particular, the plate 70 has an interior surface 90 and an exterior surface 92, and the dome 72 has an interior surface 94 and an exterior surface 96. According to the illustrated embodiment, the interior surface 80 of the housing 52 is defined by the interior surfaces 90, 94 of the plate 70 and the dome 72, while the exterior surface 84 of the housing 52 is defined by the exterior surfaces 92, 96 of the plate 70 and dome 72.

As noted above, the housing 52 is attached to the skin of a patient. In particular, an adhesive may be used. The adhesive can be adapted to releasably secure the housing 52 to a patient's skin during a single application or can be configured to adhere for a longer period, such as with a delayed delivery operation. The adhesive is disposed in a layer 100 on a portion 102 of the exterior surface 84 of the housing 52, and in particular on the exterior surface 92 of the plate 70. The adhesive layer 100 can be covered with a removable, disposable sheet 104 (shown in Fig. 1) prior to application of the housing 52 to the skin of the wearer. In this embodiment of the drug delivery device 50, the exterior surface 92 of the plate 70 defines a distal surface 105, which is the actual surface of the drug delivery device 50 that is attached to the patient 101 by the adhesive layer 100, as shown in Fig. 2. The plate 70 further defines an opening 107 extending therethrough for drug delivery, as will be described in more detail below.

Turning now to details of the operation of the device 50 as shown in Fig. 2, a cannula 54, a needle insertion mechanism 56, and a delivery mechanism 150 are disposed within the housing 52 and operably coupled to a controller 60. The controller 60 operates the needle insertion mechanism 56 to move the cannula 54 and operates the delivery mechanism 150 to deliver a drug 153 from a primary container 62 to the patient 101.

The drug delivery device 50, and the cannula 54 thereof, can have a storage state (not shown) where the cannula 54 occupies a retracted position with a terminal end 58 concealed inside the housing 52. The drug delivery device 50, and the cannula 54 thereof, can also have a delivery state (Fig. 2) where the cannula 54 occupies an extended position (shown in Figs. 2) where the terminal end 58 extends out of (e.g., projects from) the housing 52. Thus, when the drug delivery device 50 is placed onto a patient 101, as in Fig. 2, and the occupies the delivery state, the terminal end 58 of the cannula 54 penetrates the skin of the patient 101 to intradermal, sub-cutaneous, intramuscular, or intravenous target sites. Examples of needle insertion mechanisms may be found in U.S. Patent Nos. 7,144,384 and 7,128,727.

According to the present disclosure, the controller 60 is configured to activate the delivery mechanism 56 in response to determining that the housing 52 has been properly placed onto the patient 101. Proper placement is determined based on the state of an electrical circuit 105 including an electrically conductive portion of the cannula 54 and at least one electrode 103 (Figs. 2 and 3) disposed on or coupled to the housing 52. So configured, when the drug delivery device 50 occupies the delivery state with the cannula 54 inserted into the patient's tissue as depicted in Fig. 2, the patient's tissue closes (e.g., completes) an electrical connection between the cannula 54 and the electrode 103 such that the controller 60 recognizes a completed electrical circuit 105.

In this embodiment, the cannula 54 can include a conductive metal needle with a pointed terminal end 58. in other embodiments, the cannula 54 can include a soft or hard catheter 120 delivered into the patient with a separate delivery needle 59. In some embodiments, the cannula 54 can be constructed of a non-conductive material such as a plastic material, which may or may not be flexible. In such embodiments, the non-conductive material may be coated or embedded with a conductive material such that the cannula 54 as a whole is electrically conductive. By another approach, the terminal end 58 of the cannula 54 can be constructed of a conductive material and a remaining portion of the exterior of the cannula 54 can be constructed of a non-conductive material. In this form, the cannula 54 can include a conductive portion extending along an internal longitudinal portion to thereby connect the conductive terminal end 58 to the components of the electrical circuit 105 within the device 50. In some embodiments, the conductive material can include carbon, copper, silver, or any other electrically conductive material. By another approach, the cannula 54 can be a plastic cannula made from electrically conductive injection molded or compression molding techniques such that the resulting cannula 54 can be fully conductive similar to a stainless steel cannula. The cannula 54 can be molded from any suitable materials. In one example, the cannula 54 can be made from polyvinyl-chloride with carbon black.

As also shown in Fig. 2, the electrode 103 is carried by (e.g., disposed on) the distal surface 105 on the exterior surface 92 of the plate 70. Moreover, as shown in Fig. 2, the plate 70 defines an opening 107 through which the cannula 54 passes when the cannula 54 occupies the extended position of the delivery state of the device 50. In the illustrated embodiment, the electrode 103, the exterior surface 92 of the plate 70, and the opening 107 reside within a plane P that is substantially orthogonal to an axis A along which the cannula 54 extends through the opening 107 and into the patient's tissue 101. According to certain embodiments, the opening 107 may be unobstructed, such that there is no impediment or obstacle to the movement of the cannula 54 through the opening 107. However, to maintain the sterility of the cannula 54 and the device's container closure integrity (CCI), a pierceable septum or a shield (not shown) may be disposed in or over the opening 107.

Further, in some embodiments, the controller 60 may determine a depth that the cannula 54 has been inserted into the patient by measuring or comparing impedance, resistance, or capacitance values of the circuit. For example, in the form discussed above, where the cannula 54 has a conductive terminal end 58, the impedance, resistance, or capacitance values for the electrical circuit 105 will change as the depth of the cannula terminal end 58 changes. Accordingly, the controller 60 can determine, with reference to expected values, a depth of the cannula terminal end 58 based on modulation of the current readings to thereby confirm and record a depth of the cannula 54 during a drug dispensing operation. In some instances, delivery depth of a drug, such as to intradermal, sub-cutaneous, intramuscular, or intravenous sites, for example, can influence pharmacokinetics, which can result in therapeutic differences for a particular drug. Utilizing devices as described herein, interested parties can confirm, with data, that the device 50 was positioned correctly and that the cannula 54 was inserted to a target depth.

In another approach, the electrode 103 can be capacitance-based so that the controller 60 can measure the capacitance of the electrode 103. With this configuration, the controller 60 can monitor the capacitance of the electrode 103 and, optionally, the cannula 54, in addition to determining when the electrical circuit 105 is closed in response to the drug delivery device 50 occupying the delivery state with the cannula 54 inserted into the patient's tissue and the cannula 54 in contact with the patient's tissue. In some versions, the capacitance measurements for both the electrode 103 and the cannula 54 can be performed by the same components to reduce the footprint and costs of the device 50.

Also according to the present disclosure, after the controller 60 operates the needle insertion mechanism 56 and simultaneously with operating the delivery mechanism 150, the controller 60 monitors the flow of the drug 153 based on signals from a flow measurement device 61 associated with a fluid flow path 63 for the drug 153. The flow measurement device 61 measures a characteristic of the flow of the drug 153 that enables the controller 60 to determine a volume of drug delivered to the patient. The volume measurement, combined with monitoring the completed electrical circuit 150, allows the controller 60 to determine whether a full dose of the drug 153 was delivered while the device 50 was correctly positioned on the patient.

Having thus described the device 50 and its use in general terms, the structure and operation of the device 50 is now described in greater detail. According to the illustrated embodiment, the primary container 62 may include the delivery mechanism 150 and a reservoir 152. According to an embodiment of the present disclosure, the reservoir 152 and delivery mechanism 150 may be defined in part by a combination of a rigid-walled cylinder 154 and a plunger 156 fitted to move along a longitudinal axis 158 of the cylinder 154. The movement of the plunger 156 may be caused by the operation of a gear train (not shown) that is connected to a motor of the delivery mechanism 150, according to one variant. Other similar mechanisms for moving the plunger along the cylinder may be found in U.S. Patent Nos. 7,144,384; 7,128,727, 6,656,159 and 6,656,158.

According to other variants, a non-rigid collapsible pouch may be substituted for the rigid-walled cylinder 154 and the plunger 156. It will be recognized that where the reservoir 152 is in the form of a non-rigid collapsible pouch, a spring-based mechanical system (not shown) may be used to compress and pressurized the reservoir 152. According to still further variants, a non-mechanical system may be used to move the plunger 156 or compress the bag. For example, a gas-generating system may be used, including a two-component system wherein the components are kept apart until the gas is to be generated, in which case they are combined. As a further alternative, a swellable gel may be used, wherein the introduction of water from a source internal to the device causes the gel to increase in dimension to move the plunger or compress the pouch. Examples of such alternative mechanisms may be found in U.S. Patent Nos. 5,957,895; 5,858,001; and 5,814,020.

As shown in Fig. 2, the fluid flow path 63 includes the reservoir 152, the cannula 54, and the conduits 65 extending therebetween, which can take any desired form, such as tubing and the like. As stated above, the flow measurement device 61 is associated with the fluid flow path 63 to thereby measure a characteristic of the flow of the drug 153. The flow measurement device 61 can take any suitable form. For example, the flow measurement device 61 can be coupled to the fluid connection 65. With this configuration, the flow measurement device 61 can include a thermal mass transport system that is configured to heat the drug 153 to a predetermined temperature and sensors configured to measure a temperature loss over a predetermined distance of the conduit 65. In other examples, the flow measurement device 61 can be a charge mass transport system, a Doppler velocimetry system utilizing Doppler shift in a laser beam to measure the velocity of the flow of the drug 153, a turbine flow meter, a pressure-drop meter, and so forth. In another example, the flow measurement device 61 can be coupled to a motor, such as an encoder, or other driver, such as the plunger 156, for forcing the drug 153 out of the reservoir 152 to thereby measure an amount of travel or compression and extrapolate an amount of drug 153 forced towards the cannula 54. In another example, the flow measurement device 61 can be coupled to capacitance sensors along the length of the reservoir 152 to thereby measure the change in capacitance of the contents of the reservoir 152 as the drug 153 is forced towards the cannula 54 and measure the volume of drug delivered. In yet another example, the flow measurement device 61 can be operably coupled to or indirectly measure the position of the plunger 156 or plunger rod as the plunger 156 is driven through the reservoir 152 to an end-of-dose position indicating that the entire volume of the drug 153 is dispensed. The flow measurement device 61 in these forms can utilize optical or magnetic measurement sensors, for example.

Further, some embodiments of the drug delivery device 50 may include a fill port 160 in fluid communication with the reservoir 152, the fill port 160 having an inlet 162 disposed on the exterior surface 84 of the housing 52. The inlet 162 may be adapted to receive a luer tip of a syringe, although a pierceable rubber septum may be used instead, for example. The fill port 160 may also include a cover disposed in the inlet 162 to close the fill port 160. An outlet 164 of the fill port 160 is connected to the reservoir 152. One or more filters may be disposed between the inlet 162 and the outlet 164 to limit the passage of air or particulate matter into the reservoir 152 along with the drug. In use, the healthcare provider may inject the drug through the fill port 160 into the reservoir 152. in alternative embodiments, the reservoir 152 of the primary container 62 of the drug delivery device 50 may be pre-filled with drug such that no fill port 160 is needed.

In addition, as shown in Fig. 2, one version of the primary container 62 may include a pinch valve 168 or other type of valve disposed between the reservoir 152 and the cannula 54. The inclusion of the valve 168 permits greater control of the timing of the delivery of the drug. Other devices, such as flow regulators, may be disposed in the flow path between the reservoir 152 and the patient to control the flow of the drug therebetween.

The controller 60 is coupled to the needle insertion mechanism 56 and the primary container 62. The controller 60 is programmed to control the needle insertion mechanism 56 and the drive mechanism 150 of the primary container 62 to carry out certain activities. The controller 60 is disposed within the sealed space 82 defined within the housing 52 and, in some versions, is programmed prior to being disposed within the sealed space 82. Thus, once the controller 60 is disposed in the space 82 and the housing 52 is sealed, the controller 60 in some versions may not be reprogrammed. In other versions, the controller 60 may be able to communicate with a remote device such as a controller, smart phone, etc., for reprogramming even after it is disposed in and sealed within the housing 52.

According to one embodiment, the controller 60 may include a programmable processor 180, a memory 182, and a power supply (not shown) coupled to the processor 180. The power supply may include one or more batteries, for example. The memory 182 can store logic (e.g., programming) that is executable by the processor 180 for operating the drug delivery device 50.

For instance, as noted above, the controller 60 is configured to determine proper placement of the drug delivery device 50 onto the patient 101 prior to and during drug delivery. The controller 60 may be programmed to perform this action by determining when the electrical circuit 105 including the cannula 54 and the electrode 103 is completed, which occurs when the electrode 103 contacts the patient's skin simultaneously with the cannula 54 being inserted into the patient 101. In this configuration, electrical current can then travel between the cannula 54 and the electrode 103 through the patient 101 to complete the circuit 105.

Once the controller 60 recognizes that the circuit 105 is completed, the controller 60 can activate the delivery mechanism 150 to drive drug from the primary container 62 through the cannula 54 and into the patient 101. If at any time during delivery the drug delivery device 50 moves out of proper positioning on the patient 101 such that the electrode 103 and/or cannula 54 loses contact with the patient 101, the electrical circuit 105 breaks (e.g., opens). The controller 60 identifies this open circuit 105 and can perform any number of actions. For example, in one embodiment, upon detecting an open circuit 105 while the delivery mechanism 150 is actively operating and delivering drug, the controller 60 may immediately inhibit operation of the delivery mechanism 150 and/or activate a notification device 192. The notification device 192 can be configured to generate a notification that alerts the user of the drug delivery device 50 of a faulty operational state. For example, the notification device 192 can generate a visible alarm (e.g., a solid light, flashing lights, etc.), an audible alarm (e.g., one or more chimes, bells, or whistles, a song, a voice recorded message, etc.), and/or a tactile alarm (e.g., vibrations, pulsing, etc.).

The controller 60 can further perform actions with regard to determinations based on measurements from the flow measurement device 61. For example, the controller 60 can monitor the volume of drug delivered and deactivate operation of the device 50 in response to determining that a full dose of the drug 153 has been delivered. The controller 60 can also activate the notification device 192 in response to determining that operation has stopped before a full dose of the drug 153 has been delivered, which is another example faulty operation state, and/or in response to determining that a full dose of the drug 153 has been delivered.

In some embodiments, the drug delivery device 50 can also include a communication module 183, as shown in Figs. 2, disposed on-board the drug delivery device 50 and in communication with the controller 60. According to one embodiment, the communication module 183 may be a Bluetooth/Bluetooth Low Energy module that is coupled to the controller 60. Alternatively, other protocols may be used by the communication module 183, such as RFID, Zigbee, Wi-Fi, NFC, radio, and others. In another example, the notification device 192 can include the controller 60 sending a signal or message to a device of the patient or other party, such as a doctor, in response to identifying the faulty operational state. This functionality allows the device 50 to accurately track drug dispensing including whether the device 50 was positioned correctly and a full dose was delivered and send this information to the patient or a third party. For example, a message can include the time and date of the operation, the amount of drug delivered, and a confirmation that the electrical circuit 105 was closed during the operation.

The controller 60 can be configured to cause the communication module 183 to transmit information to a remote computing device (e.g., a remote computer, tablet, smart phone, etc.), where that information regards the status of the electrical circuit 105 and/or the flow measurement device 61. For example, upon detecting the electrical circuit 105 being closed by proper placement of the drug delivery device 50 on the patient 101, the controller 106 can be configured to send a signal via the communication module 183 to a remote computing device indicating that the device 50 is ready for use. Similarly, if proper placement is disturbed during drug delivery, the controller 60 can be configured to send a signal via the communication module 183 to a remote computing device indicating that the device 50 operation has been compromised and/or terminated. Then once the device 50 is returned to proper placement, the controller 60 can be configured to send a signal via the communication module 183 to a remote computing device indicating that the device 50 is again properly positioned. The controller 60 can also monitor the volume of drug delivered and can be configured to send a signal via the communication module 183 in response to determining that a full dose of the drug 153 has been delivered, in response to determining that operation has stopped before a full dose of the drug 153 has been delivered, and/or in response to determining that a volume of drug was delivered without the electrical circuit 105 being closed. In any of these situations, the signal can also include the amount of drug delivered.

In embodiments where the remote computing device is a smart phone or tablet in the possession of the user, for example, the controller 60 can send the foregoing messages in text format, graphical format, etc. Additionally, in some embodiments, the remote computing device can save the various communications received from the communication module 183 to allow further processing to determine patient compliance and other usage information.

As discussed, the controller 60 is programmed to determine when the drug delivery device 50 is properly placed onto the patient 101. But prior to this determination, the controller 60 must first be activated itself. A number of different mechanisms may be used to first activate the controller 60 immediately after placement onto the patient 101. According to an exemplary embodiment, the drug delivery device 50 may include an activation button 184 (shown in Fig. 2) coupled to the controller 60. The button 184 may be disposed so that it depends through the exterior surface 84 of the housing 52, and the controller 60 may be responsive to actuation of the button 184 (e.g., depression of the button 184) to initiate the logic stored on the memory 182.

Thus, once the controller 60 is activated, it can initiate the needle insertion mechanism 56 to move the cannula 54 from the retracted state to the deployed state. Then, the controller 60 can determine whether the drug delivery device 50 has been properly placed, and if so, activate the delivery mechanism 150 and proceed as discussed above. The controller 60 can then monitor the volume of drug delivered to the patient and, upon the completion of the entire drug delivery process, the controller 60 can initiate the needle insertion mechanism 56 to retract the cannula 54 back into the housing 52 prior to the user detaching the drug delivery device 50 from the patient 101.

While the foregoing discussion relates to a wearable style on-body injector incorporating the principles of the present disclosure, one other version of the disclosure can include an autoinjector type drug delivery device.

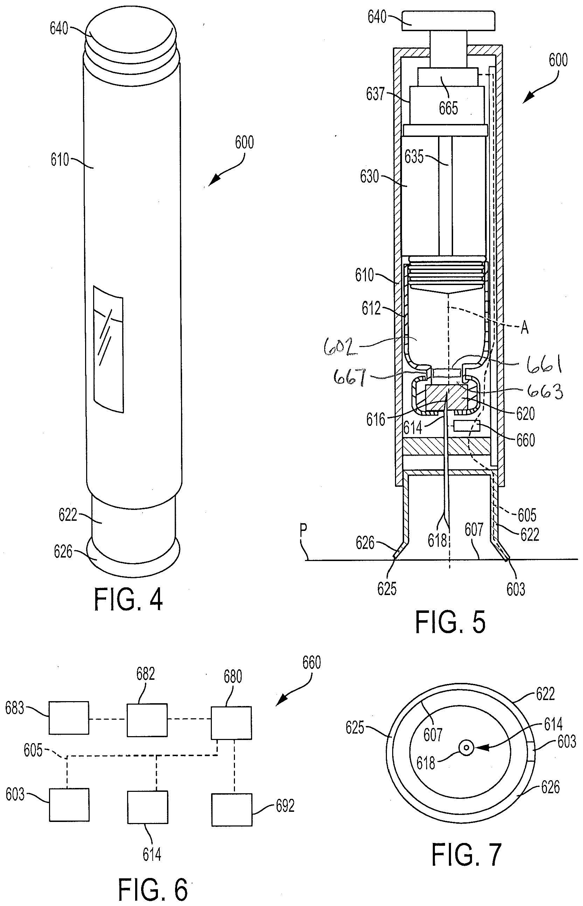

Figs. 4-6 depict an example autoinjector 600 incorporating the principles of the present disclosure and including a housing 610 in which may be disposed assemblies or structures that insert or enable insertion of a cannula 614 into the patient (not shown), and that inject a drug 602 from a primary container 612 through the cannula 614 and into the patient.

In some embodiments, the cannula 614 has a first end 616 that may be connected or connectable in fluid communication to the primary container 612 and a second end 618 that may be inserted into the patient. The cannula 614 may be, for example, a conductive metal needle sized such that the second end 618 is received under the skin so as to deliver a subcutaneous injection of the drug within the primary container 612. In other embodiments, the cannula 614 can be constructed of a non-conductive material with a conductive material coating or embedded therein, as described above with reference to the embodiment depicted in Figs. 1-3. By another approach, the second end 618 of the cannula 614 can be constructed of a conductive material and a remaining portion of the exterior of the cannula 614 can be constructed of a non-conductive material. In this form, the cannula 614 can include a conductive portion extending along an internal longitudinal portion to thereby connect the conductive second end 618 to other components within the device 600. In some embodiments, the conductive material can include carbon, copper, silver, or any other electrically conductive material.

The first end 616 of the cannula 614 may be disposed through a wall 620 of the primary container 612, and thus be connected in fluid communication with the primary container 612. As illustrated, the first end 616 of the needle 614 may be disposed only partially through the wall 620 (which wall 620 may be a resealable septum or stopper, for example) such that the first end 616 of the cannula 614 may not be connected in fluid communication with the primary container 612 until the second end 618 is inserted into the patient. So configured, the wall 620 of this embodiment serves as a lock that maintains sterility of the primary container 612. Moreover, in some embodiments, once removed from the patient, the first end 616 may again become disconnected from fluid communication with the primary container 612. In such a circumstance, the first end 616 may be described as connectable in fluid communication with the primary container 612, although it will be recognized that there are other mechanisms by which the first end 616 of the cannula 614 may be connectable, but not connected, in fluid communication with the primary container 612.

In the disclosed embodiment, the drug delivery device 600 includes a needle guard 622 to limit access to the second end 618 of the cannula 614 when the drug delivery device 600 is not in use. According to certain embodiments, the needle guard 622 may have a biasing element (not shown) that urges the needle guard 622 away from the housing 610 and into a protracted position, as shown in Fig. 5, such that a distal end 626 of the needle guard 622 extends beyond the second end 618 of the cannula 614 until if and when the cannula 614 is inserted into a patient. In fact, the injection of the cannula 614 may be actuated according to certain embodiments of the autoinjector 600 by disposing the distal end 626 of the needle guard 622 on or against the skin of the patient and applying a downward force.

According to certain embodiments, the distal end 626 of the needle guard 622 defines a distal surface 625 positioned in contact with the skin of the patient when the drug delivery device 600 is in use. As shown in Fig. 5, the distal surface 625 and an opening 607 of the needle guard 622 reside within a plane P that is substantially orthogonal to an axis A along which the cannula 614 extends when passing through the opening 607.

In some embodiments, the drug delivery device 600 includes at least one delivery mechanism 630 that may be used to inject the drug from the primary container 612 through the cannula 614 and into the patient. The delivery mechanism 630 may include a plunger 635 driven by a motor 637 activated by an actuator 640. In other embodiments, the delivery mechanism 630 may include one or more springs, a source of pressurized gas or a source of a material that undergoes a phase change, such that the escaping gas or phase changing material that provides a motive force that may be applied to the primary container 612 to eject the drug therefrom. According to still other embodiments, the delivery mechanism 630 may include any other electromechanical system, for example.

The autoinjector 600 may further include a controller 660. As shown in Fig. 6, the controller 660 can include a processor 680 and a memory 682 storing logic that is executable by the processor 680. The processor 680 is electrically connected to, amongst other things, the cannula 614 and an electrode 603 disposed on the distal surface 625 of the needle guard 622. The controller 660 may also be coupled to a power supply, e.g. a battery, (not shown) and can include a notification device 692 and a communication module 683 for performing notification and communication actions similar to those described above with respect to the embodiment depicted in Figs. 1-3. Finally, as shown in Fig. 5, the controller 660 can be coupled to the actuator 640.

The processor 680 may be programmed to carry out certain actions that the controller 660 is adapted to perform and the memory 682 may include one or more tangible non-transitory readable memories having logic (e.g., executable instructions) stored thereon, which instructions when executed by the processor 680 may cause the at least one processor 680 to carry out the actions that the controller 660 is adapted to perform. Additionally, the controller 660 may include other circuitry for carrying out certain actions in accordance with the principles of the present disclosure.

Based on the foregoing, the drug delivery device 600 in Figs. 4-7 can be described as having a storage state, shown in Fig. 5, and a delivery state, which is not shown. In the storage state, the cannula 614 occupies a retracted position concealed within the protracted needle guard 622 of the housing 610. In the delivery state, as will be described, the cannula 614 occupies an extended position where its terminal end (i.e., the second end 618) extends out through the opening 607 of the needle guard 622 which occupies a retracted position. Accordingly, during use, and with the drug delivery device 600 occupying the storage state, the distal surface 625 of the needle guard 622 is placed against a patient's skin. A force is applied to the autoinjector 600 in a direction toward the patient to inject the cannula 614 into the patient. That is, as the force is applied, a counter force urges the needle guard 622 to retract into the housing 610 thereby allowing the second end 618 of the cannula 614 to extend out of the opening 607 and beyond the distal surface 625 of the needle guard 622 and into the patient. Simultaneously, with the version depicted in Fig. 5, the first end 616 of the cannula 614 fully penetrates the wall 620 to become in direct fluid communication with the reservoir 612. Once the cannula 614 is injected into the patient, both the cannula 614 and the electrode 603 disposed on the distal surface 625 of the needle guard 622 are in contact with the patient and an electrical circuit 605 can be completed through the patient's tissue.

As with the embodiment described above with reference to Figs. 1-3, the controller 660 recognizes when the circuit 605 is completed and enables activation of the delivery mechanism 630. In the disclosed version of the autoinjector 600, the controller 660 is in communication with a lockout device 665 which is part of the actuator 640. The lockout device 665 can include an electrical switch, for example, that prohibits actuation of the motor 637 for driving the plunger 635 until the controller 660 sends a signal to unlock the lockout device 665. As such, when the controller 660 recognizes that the circuit 605 is complete, indicating proper placement of the drug delivery device 600, the controller 660 unlocks the lockout device 665 which in turn allows the user to depress the actuator 640 and activate the motor 637 to drive the plunger 635 of the delivery mechanism 630. If at any point during operation of the delivery mechanism 630, the controller 660 determines that the electrical circuit 605 becomes broken, indicating that the proper placement of the delivery device 600 has been disturbed, the controller 660 can send a signal to terminate operation of the delivery mechanism 630. In embodiments where the delivery mechanism 630 includes the motor 637 and plunger 635, the controller 660 can terminate operation by re-locking the lockout device 665, for example. In other versions, where the delivery mechanism 630 can include a spring or other mechanical or pneumatic pressure source, the lockout device 665 may include a mechanical lever operated by a solenoid valve or other electro-mechanical drive such that the mechanical lever physically interferes with movement of the plunger 635 upon actuation to stop advancement of the plunger 635.

Further, in some embodiments, the controller 660 may determine a depth that the cannula 614 has been inserted into the patient by measuring or comparing impedance, resistance, or capacitance values of the circuit. For example, in the form discussed above, where the cannula 614 has a conductive second end 618, the impedance, resistance, or capacitance values for the electrical circuit 605 will change as the depth of the cannula second end 618 changes. Accordingly, the controller 660 can determine, with reference to expected values, a depth of the cannula second end 618 based on modulation of current readings to thereby confirm and record a depth of the cannula 614 during a drug dispensing operation

Also according to the present disclosure, the controller 660 monitors the flow of the drug 602 through the device 600 based on signals from a flow measurement device 661 associated with a fluid flow path 663 for the drug 602. The flow measurement device 661 measures a characteristic of the flow of the drug 602 that enables the controller 660 to determine a volume of drug delivered to the patient. The volume measurement, combined with monitoring the completed electrical circuit 605, allows the controller 660 to determine whether a full dose of the drug 602 was delivered while the device 600 was correctly positioned on the patient.

As stated above, the flow measurement device 661 is associated with the fluid flow path 663 to thereby measure a characteristic of the flow of the drug 602. The flow measurement device 661 can take any suitable form. For example, the flow measurement device 661 can be coupled to a throat 667 or other portion of the primary container 612 or to the cannula 614. With this configuration, the flow measurement device 661 can include a thermal mass transport system that is configured to heat the drug 602 to a predetermined temperature and sensors configured to measure a temperature loss over a predetermined distance of the fluid flow path 663. In other examples, the flow measurement device 661 can be a charge mass transport system, a Doppler velocimetry system utilizing Doppler shift in a laser beam to measure the velocity of the flow of the drug 602, a turbine flow meter, a pressure-drop meter, and so forth. In another example, the flow measurement device 661 can be coupled to the motor 637, such as an encoder, or other driver, such as the plunger 635, for forcing the drug 602 out of the reservoir primary container 612 to thereby measure an amount of travel or compression and extrapolate an amount of drug 602 forced towards the cannula 614. In another example, the flow measurement device 661 can be coupled to capacitance sensors along the length of the reservoir 612 to thereby measure the change in capacitance of the contents of the reservoir 612 as the drug 602 is forced towards the cannula 614 and measure the volume of drug delivered.

In addition to inhibiting delivery of the drug, the controller 660 can further activate the notification device 692, similar to that described with reference to the embodiment depicted in Figs. 1-3. The notification device 692 can be configured to generate a notification that alerts the user of the drug delivery device 600 of a faulty operational state. For example, the notification device 692 can generate a visible alarm (e.g., a solid light, flashing lights, etc.), an audible alarm (e.g., one or more chimes, bells, or whistles, a song, a voice recorded message, etc.), and/or a tactile alarm (e.g., vibrations, pulsing, etc.). In some embodiments, the controller 660 can also activate the communication module 683. According to one embodiment, the communication module 683 may be a Bluetooth/Bluetooth Low Energy module that is coupled to the controller 660. Alternatively, other protocols may be used by the communication module 683, such as RFID, Zigbee, Wi-Fi, NFC, radio, and others.

In another example, the notification device 692 can include the controller 660 sending a signal or message to a device of the patient or other party, such as a doctor, in response to identifying a faulty operational state. This functionality allows the device 600 to accurately track drug dispensing including whether the device 600 was positioned correctly and a full dose was delivered and send this information to the patient or a third party. For example, a message can include the time and date of the operation, the amount of drug delivered, a confirmation that the electrical circuit 605 was closed during the operation, and/or a depth of the second end 618 of the cannula 614.

The controller 660 can further perform actions with regard to determinations based on measurements from the flow measurement device 661. For example, the controller 660 can monitor the volume of drug delivered and deactivate operation of the device 600 in response to determining that a full dose of the drug 602 has been delivered. The controller 660 can also activate the notification device 692 in response to determining that operation has stopped before a full dose of the drug 602 has been delivered, which is another example faulty operation state, and/or in response to determining that a full dose of the drug 602 has been delivered

The controller 660 can be configured to cause the communication module 683 to transmit information to a remote computing device (e.g., a remote computer, tablet, smart phone, etc.), where that information regards the status of the electrical circuit 605. For example, upon detecting the electrical circuit 605 being closed by proper placement of the drug delivery device 600 on the patient, the controller 660 can be configured to send a signal via the communication module 683 to a remote computing device indicating that the device 600 is ready for use. Similarly, if proper placement is disturbed during drug delivery, the controller 660 can be configured to send a signal via the communication module 683 to a remote computing device indicating that the device 600 operation has been compromised and/or terminated. Then once the device 600 is returned to proper placement on the patient, the controller 660 can be configured to send a signal via the communication module 683 to a remote computing device indicating that the device 660 is again properly positioned.

The controller 660 can also monitor the volume of drug delivered and can be configured to send a signal via the communication module 683 in response to determining that a full dose of the drug 602 has been delivered, in response to determining that operation has stopped before a full dose of the drug 602 has been delivered, and/or in response to determining that a volume of drug was delivered without the electrical circuit 605 being closed. In any of these situations, the signal can also include the amount of drug delivered.

In embodiments where the remote computing device is a smart phone or tablet, for example, the controller 660 can send the foregoing messages in text format, graphical format, etc. Additionally, in some embodiments, the remote computing device can save the various communications received from the communication module 183 to allow further processing to determine patient compliance and other usage information.

While the foregoing drug delivery devices 50, 600 have been described as including a single electrode 103, 603 disposed on the distal surfaces 102, 625 of the respective housings 52, 610, other embodiments could have a more than one. For example, in some alternative embodiments, the distal surface of a housing of a drug delivery device constructed in accordance with the principles of the present application may have two, three, four, five, six, seven, eight, nine, ten, or any number of second electrodes. In some embodiments including more than a single second electrode on the distal surface, the electric circuit can be considered complete (e.g., closed) when at least one electrode contacts the patient simultaneously with the cannula inserted into the patient. In other embodiments including more than a single second electrode on the distal surface, the electric circuit can be considered complete (e.g., closed) when only all of the second electrodes are in contact the patient simultaneously with the cannula inserted into the patient.

Additionally, while the drug delivery devices 50, 600 thus far described include single cannulas for drug delivery, the present disclosure also applies to drug deliveries that include multiple cannulas. For example, one alternative drug delivery device can include a plurality of micro-cannulas, which can often be referred to as micro-needles, for drug delivery. In such an embodiment, at least one of the micro-cannulas can be electrically conductive. In some embodiments, all of the micro-cannulas can be electrically conductive.

The above description describes various assemblies, devices, and methods for use with a drug delivery device. It should be clear that the system, drug delivery device or methods can further comprise use of a medicament listed below with the caveat that the following list should neither be considered to be all inclusive nor limiting. The medicament will be contained in a reservoir. In some instances, the reservoir is a primary container that is either filled or pre-filled for treatment with the medicament. The primary container can be a cartridge or a pre-filled syringe.

For example, the drug delivery device or more specifically the reservoir of the device may be filled with colony stimulating factors, such as granulocyte colony-stimulating factor (G-CSF). Such G-CSF agents include, but are not limited to, Neupogen^{®} (filgrastim) and Neulasta^{®} (pegfilgrastim). In various other embodiments, the drug delivery device may be used with various pharmaceutical products, such as an erythropoiesis stimulating agent (ESA), which may be in a liquid or a lyophilized form. An ESA is any molecule that stimulates erythropoiesis, such as Epogen^{®} (epoetin alfa), Aranesp^{®} (darbepoetin alfa), Dynepo^{®} (epoetin delta), Mircera^{®} (methyoxy polyethylene glycol-epoetin beta), Hematide^{®}, MRK-2578, INS-22, Retacrit^{®} (epoetin zeta), Neorecormon^{®} (epoetin beta), Silapo^{®} (epoetin zeta), Binocrit^{®} (epoetin alfa), epoetin alfa Hexal, Abseamed^{®} (epoetin alfa), Ratioepo^{®} (epoetin theta), Eporatio^{®} (epoetin theta), Biopoin^{®} (epoetin theta), epoetin alfa, epoetin beta, epoetin zeta, epoetin theta, and epoetin delta, as well as the molecules or variants or analogs thereof as disclosed in the following patents or patent applications: U.S. Patent Nos. 4,703,008; 5,441,868; 5,547,933; 5,618,698; 5,621,080; 5,756,349; 5,767,078; 5,773,569; 5,955,422; 5,986,047; 6,583,272; 7,084,245; and 7,271,689; and PCT Publication Nos. WO 91/05867; WO 95/05465; WO 96/40772; WO 00/24893; WO 01/81405; and WO 2007/136752.

An ESA can be an erythropoiesis stimulating protein. As used herein, "erythropoiesis stimulating protein" means any protein that directly or indirectly causes activation of the erythropoietin receptor, for example, by binding to and causing dimerization of the receptor. Erythropoiesis stimulating proteins include erythropoietin and variants, analogs, or derivatives thereof that bind to and activate erythropoietin receptor; antibodies that bind to erythropoietin receptor and activate the receptor; or peptides that bind to and activate erythropoietin receptor. Erythropoiesis stimulating proteins include, but are not limited to, epoetin alfa, epoetin beta, epoetin delta, epoetin omega, epoetin iota, epoetin zeta, and analogs thereof, pegylated erythropoietin, carbamylated erythropoietin, mimetic peptides (including EMP1/hematide), and mimetic antibodies. Exemplary erythropoiesis stimulating proteins include erythropoietin, darbepoetin, erythropoietin agonist variants, and peptides or antibodies that bind and activate erythropoietin receptor (and include compounds reported in U.S. Publication Nos. 2003/0215444 and 2006/0040858) as well as erythropoietin molecules or variants or analogs thereof as disclosed in the following patents or patent applications: U.S. Patent Nos. 4,703,008; 5,441,868; 5,547,933; 5,618,698; 5,621,080; 5,756,349; 5,767,078; 5,773,569; 5,955,422; 5,830,851; 5,856,298; 5,986,047; 6,030,086; 6,310,078; 6,391,633; 6,583,272; 6,586,398; 6,900,292; 6,750,369; 7,030,226; 7,084,245; and 7,217,689; U.S. Publication Nos. 2002/0155998; 2003/0077753; 2003/0082749; 2003/0143202; 2004/0009902; 2004/0071694; 2004/0091961; 2004/0143857; 2004/0157293; 2004/0175379; 2004/0175824; 2004/0229318; 2004/0248815; 2004/0266690; 2005/0019914; 2005/0026834; 2005/0096461; 2005/0107297; 2005/0107591; 2005/0124045; 2005/0124564; 2005/0137329; 2005/0142642; 2005/0143292; 2005/0153879; 2005/0158822; 2005/0158832; 2005/0170457; 2005/0181359; 2005/0181482; 2005/0192211; 2005/0202538; 2005/0227289; 2005/0244409; 2006/0088906; and 2006/0111279; and PCT Publication Nos. WO 91/05867; WO 95/05465; WO 99/66054; WO 00/24893; WO 01/81405; WO 00/61637; WO 01/36489; WO 02/014356; WO 02/19963; WO 02/20034; WO 02/49673; WO 02/085940; WO 03/029291; WO 2003/055526; WO 2003/084477; WO 2003/094858; WO 2004/002417; WO 2004/002424; WO 2004/009627; WO 2004/024761; WO 2004/033651; WO 2004/035603; WO 2004/043382; WO 2004/101600; WO 2004/101606; WO 2004/101611; WO 2004/106373; WO 2004/018667; WO 2005/001025; WO 2005/001136; WO 2005/021579; WO 2005/025606; WO 2005/032460; WO 2005/051327; WO 2005/063808; WO 2005/063809; WO 2005/070451; WO 2005/081687; WO 2005/084711; WO 2005/103076; WO 2005/100403; WO 2005/092369; WO 2006/50959; WO 2006/02646; and WO 2006/29094.

Examples of other pharmaceutical products for use with the device may include, but are not limited to, antibodies such as Vectibix^{®} (panitumumab), Xgeva^{™} (denosumab) and Prolia^{™} (denosamab); other biological agents such as Enbrel^{®} (etanercept, TNF-receptor /Fc fusion protein, TNF blocker), Neulasta^{®} (pegfilgrastim, pegylated filgastrim, pegylated G-CSF, pegylated hu-Met-G-CSF), Neupogen^{®} (filgrastim, G-CSF, hu-MetG-CSF), and Nplate^{®} (romiplostim); small molecule drugs such as Sensipar^{®} (cinacalcet). The device may also be used with a therapeutic antibody, a polypeptide, a protein or other chemical, such as an iron, for example, ferumoxytol, iron dextrans, ferric glyconate, and iron sucrose. The pharmaceutical product may be in liquid form, or reconstituted from lyophilized form.

Among particular illustrative proteins are the specific proteins set forth below, including fusions, fragments, analogs, variants or derivatives thereof:
OPGL specific antibodies, peptibodies, and related proteins, and the like (also referred to as RANKL specific antibodies, peptibodies and the like), including fully humanized and human OPGL specific antibodies, particularly fully humanized monoclonal antibodies, including but not limited to the antibodies described in PCT Publication No. WO 03/002713, as to OPGL specific antibodies and antibody related proteins, particularly those having the sequences set forth therein, particularly, but not limited to, those denoted therein: 9H7; 18B2; 2D8; 2E11; 16E1; and 22B3, including the OPGL specific antibodies having either the light chain of sequence identification number:2 as set forth therein in Figure 2 and/or the heavy chain of sequence identification number:4, as set forth therein in Figure 4, each of which is disclosed in the foregoing publication;

Myostatin binding proteins, peptibodies, and related proteins, and the like, including myostatin specific peptibodies, particularly those described in U.S. Publication No. 2004/0181033 and PCT Publication No. WO 2004/058988, particularly in parts pertinent to myostatin specific peptibodies, including but not limited to peptibodies of the mTN8-19 family, including those of sequence identification number:305-351, including TN8-19-1 through TN8-19-40, TN8-19 con1 and TN8-19 con2; peptibodies of the mL2 family of sequence identification number:357-383; the mL15 family of sequence identification number:384-409; the mL17 family of sequence identification number:410-438; the mL20 family of sequence identification number:439-446; the mL21 family of sequence identification number:447-452; the mL24 family of sequence identification number:453-454; and those of sequence identification number:615-631, each of which is disclosed in the foregoing publication;

IL-4 receptor specific antibodies, peptibodies, and related proteins, and the like, particularly those that inhibit activities mediated by binding of IL-4 and/or IL-13 to the receptor, including those described in PCT Publication No. WO 2005/047331 or PCT Application No. PCT/US2004/37242 and in U.S. Publication No. 2005/112694, particularly in parts pertinent to IL-4 receptor specific antibodies, particularly such antibodies as are described therein, particularly, and without limitation, those designated therein: L1H1; L1H2; L1H3; L1H4; L1H5; L1H6; L1H7; L1H8; L1H9; L1H10; L1H11; L2H1; L2H2; L2H3; L2H4; L2H5; L2H6; L2H7; L2H8; L2H9; L2H10; L2H11; L2H12; L2H13; L2H14; L3H1; L4H1; L5H1; L6H1, each of which is disclosed in the foregoing publication;

Interleukin 1-receptor 1 ("IL1-R1") specific antibodies, peptibodies, and related proteins, and the like, including but not limited to those described in U.S. Publication No. 2004/097712, in parts pertinent to IL1-R1 specific binding proteins, monoclonal antibodies in particular, especially, without limitation, those designated therein: 15CA, 26F5, 27F2, 24E12, and 10H7, each of which is disclosed in the aforementioned publication;

Ang2 specific antibodies, peptibodies, and related proteins, and the like, including but not limited to those described in PCT Publication No. WO 03/057134 and U.S. Publication No. 2003/0229023, particularly in parts pertinent to Ang2 specific antibodies and peptibodies and the like, especially those of sequences described therein and including but not limited to: L1(N); L1(N) WT; L1(N) 1K WT; 2xL1(N); 2xL1(N) WT; Con4 (N), Con4 (N) 1K WT, 2xCon4 (N) 1K; L1C; L1C 1K; 2xL1C; Con4C; Con4C 1K; 2xCon4C 1K; Con4-L1 (N); Con4-L1C; TN-12-9 (N); C17 (N); TN8-8(N); TN8-14 (N); Con 1 (N), also including anti-Ang 2 antibodies and formulations such as those described in PCT Publication No. WO 2003/030833, particularly Ab526; Ab528; Ab531; Ab533; Ab535; Ab536; Ab537; Ab540; Ab543; Ab544; Ab545; Ab546; A551; Ab553; Ab555; Ab558; Ab559; Ab565; AbF1AbFD; AbFE; AbFJ; AbFK; AbG1D4; AbGC1E8; AbH1C12; AbIA1; AbIF; AbIK, AblP; and AbIP, in their various permutations as described therein, each of which is disclosed in the foregoing publication;

NGF specific antibodies, peptibodies, and related proteins, and the like including, in particular, but not limited to those described in U.S. Publication No. 2005/0074821 and U.S. Patent No. 6,919,426, particularly as to NGF-specific antibodies and related proteins in this regard, including in particular, but not limited to, the NGF-specific antibodies therein designated 4D4, 4G6, 6H9, 7H2, 14D10 and 14D11, each of which is disclosed in the foregoing publication;

CD22 specific antibodies, peptibodies, and related proteins, and the like, such as those described in U.S. Patent No. 5,789,554, as to CD22 specific antibodies and related proteins, particularly human CD22 specific antibodies, such as but not limited to humanized and fully human antibodies, including but not limited to humanized and fully human monoclonal antibodies, particularly including but not limited to human CD22 specific IgG antibodies, such as, for instance, a dimer of a human-mouse monoclonal hLL2 gamma-chain disulfide linked to a human-mouse monoclonal hLL2 kappa-chain, including, but limited to, for example, the human CD22 specific fully humanized antibody in Epratuzumab, CAS registry number 501423-23-0;

IGF-1 receptor specific antibodies, peptibodies, and related proteins, and the like, such as those described in PCT Publication No. WO 06/069202, as to IGF-1 receptor specific antibodies and related proteins, including but not limited to the IGF-1 specific antibodies therein designated L1H1, L2H2, L3H3, L4H4, L5H5, L6H6, L7H7, L8H8, L9H9, L10H10, L11H11, L12H12, L13H13, L14H14, L15H15, L16H16, L17H17, L18H18, L19H19, L20H20, L21H21, L22H22, L23H23, L24H24, L25H25, L26H26, L27H27, L28H28, L29H29, L30H30, L31H31, L32H32, L33H33, L34H34, L35H35, L36H36, L37H37, L38H38, L39H39, L40H40, L41H41, L42H42, L43H43, L44H44, L45H45, L46H46, L47H47, L48H48, L49H49, L50H50, L51H51, L52H52, and IGF-1R-binding fragments and derivatives thereof, each of which is disclosed in the foregoing publication;

Also among non-limiting examples of anti-IGF-1R antibodies for use in the methods and compositions of the present invention are each and all of those described in:
(i) U.S. Publication No. 2006/0040358 (published February 23, 2006), 2005/0008642 (published January 13, 2005), 2004/0228859 (published November 18, 2004), including but not limited to, for instance, antibody 1A (DSMZ Deposit No. DSM ACC 2586), antibody 8 (DSMZ Deposit No. DSM ACC 2589), antibody 23 (DSMZ Deposit No. DSM ACC 2588) and antibody 18 as described therein;
(ii) PCT Publication No. WO 06/138729 (published December 28, 2006) and WO 05/016970 (published February 24, 2005), and Lu et al. (2004), J. Biol. Chem. 279:2856-2865, including but not limited to antibodies 2F8, A12, and IMC-A12 as described therein;
(iii) PCT Publication No. WO 07/012614 (published February 1, 2007), WO 07/000328 (published January 4, 2007), WO 06/013472 (published February 9, 2006), WO 05/058967 (published June 30, 2005), and WO 03/059951 (published July 24, 2003);
(iv) U.S. Publication No. 2005/0084906 (published April 21, 2005), including but not limited to antibody 7C10, chimaeric antibody C7C10, antibody h7C10, antibody 7H2M, chimaeric antibody *7C10, antibody GM 607, humanized antibody 7C10 version 1, humanized antibody 7C10 version 2, humanized antibody 7C10 version 3, and antibody 7H2HM, as described therein;
(v) U.S. Publication Nos. 2005/0249728 (published November 10, 2005), 2005/0186203 (published August 25, 2005), 2004/0265307 (published December 30, 2004), and 2003/0235582 (published December 25, 2003) and Maloney et al. (2003), Cancer Res. 63:5073-5083, including but not limited to antibody EM164, resurfaced EM164, humanized EM164, huEM164 v1.0, huEM164 v1.1, huEM164 v1.2, and huEM164 v1.3 as described therein;
(vi) U.S. Patent No. 7,037,498 (issued May 2, 2006), U.S. Publication Nos. 2005/0244408 (published November 30, 2005) and 2004/0086503 (published May 6, 2004), and Cohen, et al. (2005), Clinical Cancer Res. 11:2063-2073, e.g., antibody CP-751,871, including but not limited to each of the antibodies produced by the hybridomas having the ATCC accession numbers PTA-2792, PTA-2788, PTA-2790, PTA-2791, PTA-2789, PTA-2793, and antibodies 2.12.1, 2.13.2, 2.14.3, 3.1.1, 4.9.2, and 4.17.3, as described therein;
(vii) U.S. Publication Nos. 2005/0136063 (published June 23, 2005) and 2004/0018191 (published January 29, 2004), including but not limited to antibody 19D12 and an antibody comprising a heavy chain encoded by a polynucleotide in plasmid 15H12/19D12 HCA (y4), deposited at the ATCC under number PTA-5214, and a light chain encoded by a polynucleotide in plasmid 15H12/19D12 LCF (κ), deposited at the ATCC under number PTA-5220, as described therein; and
(viii) U.S. Publication No. 2004/0202655 (published October 14, 2004), including but not limited to antibodies PINT-6A1, PINT-7A2, PINT-7A4, PINT-7A5, PINT-7A6, PINT-8A1, PINT-9A2, PINT-11A1, PINT-11A2, PINT-11A3, PINT-11A4, PINT-11A5, PINT-11A7, PINT-11A12, PINT-12A1, PINT-12A2, PINT-12A3, PINT-12A4, and PINT-12A5, as described therein; each and all of which are herein incorporated by reference in their entireties, particularly as to the aforementioned antibodies, peptibodies, and related proteins and the like that target IGF-1 receptors;

B-7 related protein 1 specific antibodies, peptibodies, related proteins and the like ("B7RP-1," also is referred to in the literature as B7H2, ICOSL, B7h, and CD275), particularly B7RP-specific fully human monoclonal IgG2 antibodies, particularly fully human IgG2 monoclonal antibody that binds an epitope in the first immunoglobulin-like domain of B7RP-1, especially those that inhibit the interaction of B7RP-1 with its natural receptor, ICOS, on activated T cells in particular, especially, in all of the foregoing regards, those disclosed in U.S. Publication No. 2008/0166352 and PCT Publication No. WO 07/011941, as to such antibodies and related proteins, including but not limited to antibodies designated therein as follow: 16H (having light chain variable and heavy chain variable sequences sequence identification number:1 and sequence identification number:7 respectively therein); 5D (having light chain variable and heavy chain variable sequences sequence identification number:2 and sequence identification number:9 respectively therein); 2H (having light chain variable and heavy chain variable sequences sequence identification number:3 and sequence identification number: 10 respectively therein); 43H (having light chain variable and heavy chain variable sequences sequence identification number:6 and sequence identification number: 14 respectively therein); 41H (having light chain variable and heavy chain variable sequences sequence identification number:5 and sequence identification number:13 respectively therein); and 15H (having light chain variable and heavy chain variable sequences sequence identification number:4 and sequence identification number: 12 respectively therein), each of which is disclosed in the foregoing publication;
IL-15 specific antibodies, peptibodies, and related proteins, and the like, such as, in particular, humanized monoclonal antibodies, particularly antibodies such as those disclosed in U.S. Publication Nos. 2003/0138421; 2003/023586; and 2004/0071702; and U.S. Patent No. 7,153,507, as to IL-15 specific antibodies and related proteins, including peptibodies, including particularly, for instance, but not limited to, HuMax IL-15 antibodies and related proteins, such as, for instance, 146B7;
IFN gamma specific antibodies, peptibodies, and related proteins and the like, especially human IFN gamma specific antibodies, particularly fully human anti-IFN gamma antibodies, such as, for instance, those described in U.S. Publication No. 2005/0004353, as to IFN gamma specific antibodies, particularly, for example, the antibodies therein designated 1118; 1118*; 1119; 1121; and 1121*. The entire sequences of the heavy and light chains of each of these antibodies, as well as the sequences of their heavy and light chain variable regions and complementarity determining regions, are each disclosed in the foregoing publication and in Thakur et al. (1999), Mol. Immunol. 36:1107-1115. In addition, description of the properties of these antibodies is provided in the foregoing publication. Specific antibodies include those having the heavy chain of sequence identification number:17 and the light chain of sequence identification number:18; those having the heavy chain variable region of sequence identification number:6 and the light chain variable region of sequence identification number:8; those having the heavy chain of sequence identification number: 19 and the light chain of sequence identification number:20; those having the heavy chain variable region of sequence identification number: 10 and the light chain variable region of sequence identification number:12; those having the heavy chain of sequence identification number:32 and the light chain of sequence identification number:20; those having the heavy chain variable region of sequence identification number:30 and the light chain variable region of sequence identification number:12; those having the heavy chain sequence of sequence identification number:21 and the light chain sequence of sequence identification number:22; those having the heavy chain variable region of sequence identification number:14 and the light chain variable region of sequence identification number:16; those having the heavy chain of sequence identification number:21 and the light chain of sequence identification number:33; and those having the heavy chain variable region of sequence identification number: 14 and the light chain variable region of sequence identification number:31, as disclosed in the foregoing publication. A specific antibody contemplated is antibody 1119 as disclosed in the foregoing U.S. publication and having a complete heavy chain of sequence identification number:17 as disclosed therein and having a complete light chain of sequence identification number: 18 as disclosed therein;
TALL-1 specific antibodies, peptibodies, and the related proteins, and the like, and other TALL specific binding proteins, such as those described in U.S. Publication Nos. 2003/0195156 and 2006/0135431, as to TALL-1 binding proteins, particularly the molecules of Tables 4 and 5B, each of which is disclosed in the foregoing publications;
Parathyroid hormone ("PTH") specific antibodies, peptibodies, and related proteins, and the like, such as those described in U.S. Patent No. 6,756,480, particularly in parts pertinent to proteins that bind PTH;
Thrombopoietin receptor ("TPO-R") specific antibodies, peptibodies, and related proteins, and the like, such as those described in U.S. Patent No. 6,835,809, particularly in parts pertinent to proteins that bind TPO-R;
Hepatocyte growth factor ("HGF") specific antibodies, peptibodies, and related proteins, and the like, including those that target the HGF/SF:cMet axis (HGF/SF:c-Met), such as the fully human monoclonal antibodies that neutralize hepatocyte growth factor/scatter (HGF/SF) described in U.S. Publication No. 2005/0118643 and PCT Publication No. WO 2005/017107, huL2G7 described in U.S. Patent No. 7,220,410 and OA-5d5 described in U.S. Patent Nos. 5,686,292 and 6,468,529 and in PCT Publication No. WO 96/38557, particularly in parts pertinent to proteins that bind HGF;
TRAIL-R2 specific antibodies, peptibodies, related proteins and the like, such as those described in U.S. Patent No. 7,521,048, particularly in parts pertinent to proteins that bind TRAIL-R2;
Activin A specific antibodies, peptibodies, related proteins, and the like, including but not limited to those described in U.S. Publication No. 2009/0234106, particularly in parts pertinent to proteins that bind Activin A;
TGF-beta specific antibodies, peptibodies, related proteins, and the like, including but not limited to those described in U.S. Patent No. 6,803,453 and U.S. Publication No. 2007/0110747, particularly in parts pertinent to proteins that bind TGF-beta;
Amyloid-beta protein specific antibodies, peptibodies, related proteins, and the like, including but not limited to those described in PCT Publication No. WO 2006/081171, particularly in parts pertinent to proteins that bind amyloid-beta proteins. One antibody contemplated is an antibody having a heavy chain variable region comprising sequence identification number:8 and a light chain variable region having sequence identification number:6 as disclosed in the foregoing publication;
c-Kit specific antibodies, peptibodies, related proteins, and the like, including but not limited to those described in U.S. Publication No. 2007/0253951, particularly in parts pertinent to proteins that bind c-Kit and/or other stem cell factor receptors;
OX40L specific antibodies, peptibodies, related proteins, and the like, including but not limited to those described in U.S. Publication No. 2006/0002929, particularly in parts pertinent to proteins that bind OX40L and/or other ligands of the OX40 receptor; and
Other exemplary proteins, including Activase^{®} (alteplase, tPA); Aranesp^{®} (darbepoetin alfa); Epogen^{®} (epoetin alfa, or erythropoietin); GLP-1, Avonex^{®} (interferon beta-1a); Bexxar^{®} (tositumomab, anti-CD22 monoclonal antibody); Betaseron^{®} (interferon-beta); Campath^{®} (alemtuzumab, anti-CD52 monoclonal antibody); Dynepo^{®} (epoetin delta); Velcade^{®} (bortezomib); MLN0002 (anti- α4β7 mAb); MLN1202 (anti-CCR2 chemokine receptor mAb); Enbrel^{®} (etanercept, TNF-receptor /Fc fusion protein, TNF blocker); Eprex^{®} (epoetin alfa); Erbitux^{®} (cetuximab, anti-EGFR / HER1 / c-ErbB-1); Genotropin^{®} (somatropin, Human Growth Hormone); Herceptin^{®} (trastuzumab, anti-HER2/neu (erbB2) receptor mAb); Humatrope^{®} (somatropin, Human Growth Hormone); Humira^{®} (adalimumab); insulin in solution; Infergen^{®} (interferon alfacon-1); Natrecor^{®} (nesiritide; recombinant human B-type natriuretic peptide (hBNP); Kineret^{®} (anakinra); Leukine^{®} (sargamostim, rhuGM-CSF); LymphoCide^{®} (epratuzumab, anti-CD22 mAb); Benlysta^{™} (lymphostat B, belimumab, anti-BlyS mAb); Metalyse^{®} (tenecteplase, t-PA analog); Mircera^{®} (methoxy polyethylene glycol-epoetin beta); Mylotarg^{®} (gemtuzumab ozogamicin); Raptiva^{®} (efalizumab); Cimzia^{®} (certolizumab pegol, CDP 870); Soliris^{™} (eculizumab); pexelizumab (anti-C5 complement); Numax^{®} (MEDI-524); Lucentis^{®} (ranibizumab); Panorex^{®} (17-1A, edrecolomab); Trabio^{®} (lerdelimumab); TheraCim hR3 (nimotuzumab); Omnitarg (pertuzumab, 2C4); Osidem^{®} (IDM-1); OvaRex^{®} (B43.13); Nuvion^{®} (visilizumab); cantuzumab mertansine (huC242-DM1); NeoRecormon^{®} (epoetin beta); Neumega^{®} (oprelvekin, human interleukin-11); Neulasta^{®} (pegylated filgastrim, pegylated G-CSF, pegylated hu-Met-G-CSF); Neupogen^{®} (filgrastim, G-CSF, hu-MetG-CSF); Orthoclone OKT3^{®} (muromonab-CD3, anti-CD3 monoclonal antibody); Procrit^{®} (epoetin alfa); Remicade^{®} (infliximab, anti-TNFa monoclonal antibody); Reopro^{®} (abciximab, anti-GP Ilb/Ilia receptor monoclonal antibody); Actemra^{®} (anti-IL6 Receptor mAb); Avastin^{®} (bevacizumab), HuMax-CD4 (zanolimumab); Rituxan^{®} (rituximab, anti-CD20 mAb); Tarceva^{®} (erlotinib); Roferon-A^{®}-(interferon alfa-2a); Simulect^{®} (basiliximab); Prexige^{®} (lumiracoxib); Synagis^{®} (palivizumab); 146B7-CHO (anti-IL15 antibody, see U.S. Patent No. 7,153,507); Tysabri^{®} (natalizumab, anti-a4integrin mAb); Valortim^{®} (MDX-1303, anti-B. anthracis protective antigen mAb); ABthrax^{™}; Vectibix^{®} (panitumumab); Xolair^{®} (omalizumab); ETI211 (anti-MRSA mAb); IL-1 trap (the Fc portion of human IgG1 and the extracellular domains of both IL-1 receptor components (the Type I receptor and receptor accessory protein)); VEGF trap (Ig domains of VEGFR1 fused to IgG1 Fc); Zenapax^{®} (daclizumab); Zenapax^{®} (daclizumab, anti-IL-2Ra mAb); Zevalin^{®} (ibritumomab tiuxetan); Zetia^{®} (ezetimibe); Orencia^{®} (atacicept, TACI-Ig); anti-CD80 monoclonal antibody (galiximab); anti-CD23 mAb (lumiliximab); BR2-Fc (huBR3 / huFc fusion protein, soluble BAFF antagonist); CNTO 148 (golimumab, anti-TNFa mAb); HGS-ETR1 (mapatumumab; human anti-TRAIL Receptor-1 mAb); HuMax-CD20 (ocrelizumab, anti-CD20 human mAb); HuMax-EGFR (zalutumumab); M200 (volociximab, anti-α5β1 integrin mAb); MDX-010 (ipilimumab, anti-CTLA-4 mAb and VEGFR-1 (IMC-18F1); anti-BR3 mAb; anti-C. difficile Toxin A and Toxin B C mAbs MDX-066 (CDA-1) and MDX-1388); anti-CD22 dsFv-PE38 conjugates (CAT-3888 and CAT-8015); anti-CD25 mAb (HuMax-TAC); anti-CD3 mAb (NI-0401); adecatumumab; anti-CD30 mAb (MDX-060); MDX-1333 (anti-IFNAR); anti-CD38 mAb (HuMax CD38); anti-CD40L mAb; anti-Cripto mAb; anti-CTGF Idiopathic Pulmonary Fibrosis Phase I Fibrogen (FG-3019); anti-CTLA4 mAb; anti-eotaxin1 mAb (CAT-213); anti-FGF8 mAb; anti-ganglioside GD2 mAb; anti-ganglioside GM2 mAb; anti-GDF-8 human mAb (MYO-029); anti-GM-CSF Receptor mAb (CAM-3001); anti-HepC mAb (HuMax HepC); anti-IFNa mAb (MEDI-545, MDX-1103); anti-IGF1R mAb; anti-IGF-1R mAb (HuMax-Inflam); anti-IL12 mAb (ABT-874); anti-IL12/IL23 mAb (CNTO 1275); anti-IL13 mAb (CAT-354); anti-IL2Ra mAb (HuMax-TAC); anti-IL5 Receptor mAb; anti-integrin receptors mAb (MDX-018, CNTO 95); anti-IP10 Ulcerative Colitis mAb (MDX-1100); anti-LLY antibody; BMS-66513; anti-Mannose Receptor/hCGβ mAb (MDX-1307); anti-mesothelin dsFv-PE38 conjugate (CAT-5001); anti-PD1mAb (MDX-1106 (ONO-4538)); anti-PDGFRa antibody (IMC-3G3); anti-TGFβ mAb (GC-1008); anti-TRAIL Receptor-2 human mAb (HGS-ETR2); anti-TWEAK mAb; anti-VEGFR/Flt-1 mAb; anti-ZP3 mAb (HuMax-ZP3); NVS Antibody #1; and NVS Antibody #2.

Also included can be a sclerostin antibody, such as but not limited to romosozumab, blosozumab, or BPS 804 (Novartis). Further included can be therapeutics such as rilotumumab, bixalomer, trebananib, ganitumab, conatumumab, motesanib diphosphate, brodalumab, vidupiprant, panitumumab, denosumab, NPLATE, PROLIA, VECTIBIX or XGEVA. Additionally, included in the device can be a monoclonal antibody (IgG) that binds human Proprotein Convertase Subtilisin/Kexin Type 9 (PCSK9). Such PCSK9 specific antibodies include, but are not limited to, Repatha^{®} (evolocumab) and Praluent^{®} (alirocumab), as well as molecules, variants, analogs or derivatives thereof as disclosed in the following patents or patent applications: U.S. Patent No. 8,030,547, U.S. Publication No. 2013/0064825, WO2008/057457, WO2008/057458, WO2008/057459, WO2008/063382, WO2008/133647, WO2009/100297, WO2009/100318, WO2011/037791, WO2011/053759, WO2011/053783, WO2008/125623, WO2011/072263, WO2009/055783, WO2012/0544438, WO2010/029513, WO2011/111007, WO2010/077854, WO2012/088313, WO2012/101251, WO2012/101252, WO2012/101253, WO2012/109530, and WO2001/031007.

Also included can be talimogene laherparepvec or another oncolytic HSV for the treatment of melanoma or other cancers. Examples of oncolytic HSV include, but are not limited to talimogene laherparepvec (U.S. Patent Nos. 7,223,593 and 7,537,924); OncoVEXGALV/CD (U.S. Pat. No. 7,981,669); OrienX010 (Lei et al. (2013), World J. Gastroenterol., 19:5138-5143); G207, 1716; NV1020; NV12023; NV1034 and NV1042 (Vargehes et al. (2002), Cancer Gene Ther., 9(12):967-978).

Also included are TIMPs. TIMPs are endogenous tissue inhibitors of metalloproteinases (TIMPs) and are important in many natural processes. TIMP-3 is expressed by various cells or and is present in the extracellular matrix; it inhibits all the major cartilage-degrading metalloproteases, and may play a role in role in many degradative diseases of connective tissue, including rheumatoid arthritis and osteoarthritis, as well as in cancer and cardiovascular conditions. The amino acid sequence of TIMP-3, and the nucleic acid sequence of a DNA that encodes TIMP-3, are disclosed in U.S. Patent No. 6,562,596, issued May 13, 2003. Description of TIMP mutations can be found in U.S. Publication No. 2014/0274874 and PCT Publication No. WO 2014/152012.

Also included are antagonistic antibodies for human calcitonin gene-related peptide (CGRP) receptor and bispecific antibody molecule that target the CGRP receptor and other headache targets. Further information concerning these molecules can be found in PCT Application No. WO 2010/075238.

Additionally, bispecific T cell engager (BiTE^{®}) antibodies, e.g. BLINCYTO^{®} (blinatumomab), can be used in the device. Alternatively, included can be an APJ large molecule agonist e.g., apelin or analogues thereof in the device. Information relating to such molecules can be found in PCT Publication No. WO 2014/099984.

In certain embodiments, the medicament comprises a therapeutically effective amount of an anti-thymic stromal lymphopoietin (TSLP) or TSLP receptor antibody. Examples of anti-TSLP antibodies that may be used in such embodiments include, but are not limited to, those described in U.S. Patent Nos. 7,982,016, and 8,232,372, and U.S. Publication No. 2009/0186022. Examples of anti-TSLP receptor antibodies include, but are not limited to, those described in U.S. Patent No. 8,101,182. In particularly preferred embodiments, the medicament comprises a therapeutically effective amount of the anti-TSLP antibody designated as A5 within U.S. Patent No. 7,982,016.

Although the drug injection device, drive damper mechanisms, systems, methods, and elements thereof, have been described in terms of exemplary embodiments, they are not limited thereto. The detailed description is to be construed as exemplary only and does not describe every possible embodiment of the invention because describing every possible embodiment would be impractical, if not impossible. Numerous alternative embodiments could be implemented, using either current technology or technology developed after the filing date of this patent that would still fall within the scope of the claims defining the invention.

It should be understood that the legal scope of the invention is defined by the words of the claims set forth at the end of this patent. The appended claims should be construed broadly to include other variants and embodiments of same, which may be made by those skilled in the art without departing from the invention, which is defined by the claims.

## Claims

1. A drug delivery device comprising:
a housing (52, 610) defining an interior cavity and a distal surface (102, 625) for contacting a patient during a dispensing operation of the drug delivery device;
a primary container (62, 612) disposed in the interior cavity of the housing and adapted to contain a drug;
a cannula (54, 614) having an electrically conductive portion, the cannula having a storage state where a terminal end (58, 618) thereof is inside the housing and a delivery state where the terminal end thereof extends out through an opening (107, 607) in the distal surface of the housing;
a fluid flow path (62, 663) for the drug including the primary container and the cannula;
a delivery mechanism (150, 630) disposed in the housing for selectively forcing the drug through the fluid flow path out of the primary container and through the cannula for delivery to a patient when the cannula occupies the delivery state;
a flow measurement device (61, 661) configured to measure a characteristic of the flow of the drug forced through the fluid flow path;
an electrode (103) disposed on the distal surface of the housing; and
a controller (60, 660) carried by the housing and electrically connected to the flow measurement device, the cannula, and the electrode, the controller configured to determine whether the drug was successfully delivered to a patient by:
determining that an electrical circuit (105, 605) including the cannula and the electrode has closed upon application of the electrode on the distal surface of the housing onto a patient's skin and insertion of the cannula into the patient; and
determining, based on the measurements of the flow measurement device and in combination with recognizing the closed electrical circuit, whether a full dose of the drug has been forced through the fluid flow path and delivered to the patient.

2. The drug delivery device of claim 1, wherein the flow measurement device (61, 661) comprises a flow sensor operably coupled to the fluid flow path (63, 663) to measure the flow of the drug therethrough.

3. The drug delivery device of claim 1 or 2, wherein the electrically conductive portion of the cannula (54, 614) is at a distal end thereof, and the controller (60, 660) is configured to determine a depth of the distal end of the cannula in the delivery state based on readings of the electrical circuit (105, 625).

4. The drug delivery device of any one of the preceding claims, further comprising a communication module (183, 683), wherein the controller (60 ,660) is configured to send a confirmation signal with the communication module in response to determining that the electrical circuit (105, 625) was closed while a full dose of the drug was dispensed.

5. The drug delivery device of claim 4, wherein the controller (60, 660) is further configured to send a fault signal with the communication module (183, 683) in response to determining that less than a full dose of the drug was dispensed or that the electrical circuit (105, 625) opened while the drug was being dispensed.

6. The drug delivery device of any one of the preceding claims, wherein the controller (60, 660) is configured to:
control operation of the delivery mechanism (150, 630); and
pause delivery of the drug in response to at least one of:
determining that the electrical circuit (105, 625) has opened before a full dose of the drug has been dispensed; or
determining that the cannula (54, 614) is at an incorrect depth.

7. The drug delivery device of any one of the preceding claims, wherein the cannula (54, 614) comprises a needle of a conductive material.

8. The drug delivery device of any one of claims 1-6, wherein the cannula (54, 614) comprises a hard or soft catheter (120).

9. The drug delivery device of claim 1, further comprising a needle insertion mechanism (56) operably coupled to the cannula (54, 614) for moving the cannula between a retracted position where the terminal end (58, 618) of the cannula is concealed in the housing (52, 610) in the storage state, and an extended position where the terminal end of the cannula extends out of the housing and beyond the distal surface (102, 625) in the delivery state.

10. The drug delivery device of claim 1, wherein the housing (52, 610) comprises a retractable needle guard (622) defining the distal surface (102, 625), the needle guard occupying a protracted position concealing the terminal end (58, 618) of the cannula (54, 614) in the storage state and a retracted position allowing the terminal end of the cannula to extend out of the housing in the delivery state.

## Patentansprüche

1. Medikamentenabgabevorrichtung, die umfasst:
ein Gehäuse (52, 610), das einen inneren Hohlraum und eine distale Oberfläche (102, 625) für den Kontakt mit einem Patienten während eines Abgabevorgangs der Medikamentenabgabevorrichtung definiert,
einen Primärbehälter (62, 612), der in dem inneren Hohlraum des Gehäuses angeordnet und dafür ausgelegt ist, ein Arzneimittel zu enthalten;
eine Kanüle (54, 614) mit einem elektrisch leitenden Abschnitt, wobei die Kanüle einen Aufbewahrungszustand, in dem sich ein Endstück (58, 618) davon im Inneren des Gehäuses befindet, und einen Abgabezustand aufweist, in dem sich das Endstück davon durch eine Öffnung (107, 607) in der distalen Oberfläche des Gehäuses nach außen erstreckt;
einen Fluiddurchflusspfad (62, 663) für das Medikament, der den Primärbehälter und die Kanüle enthält,
einen Abgabemechanismus (150, 630), der in dem Gehäuse angeordnet ist, um das Medikament selektiv durch den Fluiddurchflusspfad aus dem Primärbehälter und durch die Kanüle zur Abgabe an einen Patienten zu drücken, wenn die Kanüle den Abgabezustand einnimmt,
eine Durchflussmessvorrichtung (61, 661), die dafür konfiguriert ist, eine Charakteristik des Durchflusses des Medikaments zu messen, das durch den Fluiddurchflusspfad gedrückt wird,
eine Elektrode (103), die an der distalen Oberfläche des Gehäuses angeordnet ist, und
einen Controller (60, 660), der von dem Gehäuse getragen wird und elektrisch mit der Durchflussmessvorrichtung, der Kanüle und der Elektrode verbunden ist, wobei der Controller dafür konfiguriert ist, durch Folgendes zu bestimmen, ob das Medikament erfolgreich an einen Patienten abgegeben wurde:
Bestimmen, dass ein elektrischer Stromkreis (105, 605), der die Kanüle und die Elektrode enthält, sich geschlossen hat, nachdem die Elektrode auf der distalen Oberfläche des Gehäuses auf der Haut eines Patienten angebracht und die Kanüle in den Patienten eingeführt wurde, und
Bestimmen, basierend auf den Messungen der Durchflussmessvorrichtung und in Kombination mit dem Erkennen des geschlossenen Stromkreises, ob eine volle Dosis des Medikaments durch den Fluiddurchflusspfad gedrückt und an den Patienten abgegeben wurde.

2. Medikamentenabgabevorrichtung nach Anspruch 1, wobei die Durchflussmessvorrichtung (61, 661) einen Durchflusssensor umfasst, der funktionsfähig mit dem Fluiddurchflusspfad (63, 663) gekoppelt ist, um den Durchfluss des Medikaments durch diesen Pfad zu messen.

3. Medikamentenabgabevorrichtung nach Anspruch 1 oder 2, wobei sich der elektrisch leitende Abschnitt der Kanüle (54, 614) an ihrem distalen Ende befindet, und der Controller (60, 660) dafür konfiguriert ist, basierend auf Ablesungen des elektrischen Stromkreises (105, 625) eine Tiefe des distalen Endes der Kanüle im Abgabezustand zu bestimmen.

4. Medikamentenabgabevorrichtung nach einem der vorhergehenden Ansprüche, die ferner ein Kommunikationsmodul (183, 683) umfasst, wobei der Controller (60, 660) dafür konfiguriert ist, ein Bestätigungssignal an das Kommunikationsmodul zu senden, wenn bestimmt wird, dass der Stromkreis (105, 625) geschlossen wurde, während eine volle Dosis des Medikaments abgegeben wurde.

5. Medikamentenabgabevorrichtung nach Anspruch 4, wobei der Controller (60, 660) ferner so konfiguriert ist, dass er ein Fehlersignal an das Kommunikationsmodul (183, 683) sendet, wenn bestimmt wird, dass weniger als eine volle Dosis des Medikaments abgegeben wurde oder dass der Stromkreis (105, 625) geöffnet wurde, während das Medikament abgegeben wurde.

6. Medikamentenabgabevorrichtung nach einem der vorhergehenden Ansprüche, wobei der Controller (60, 660) für Folgendes konfiguriert ist:
Steuern des Betriebs des Abgabemechanismus (150, 630); und
Unterbrechen der Abgabe des Medikaments als Reaktion auf mindestens eines der Folgenden:
Bestimmen, dass sich der Stromkreis (105, 625) geöffnet hat, bevor eine volle Dosis des Medikaments abgegeben wurde; oder
Bestimmen, dass sich die Kanüle (54, 614) in einer falschen Tiefe befindet.

7. Medikamentenabgabevorrichtung nach einem der vorhergehenden Ansprüche, wobei die Kanüle (54, 614) eine Nadel aus einem leitfähigen Material umfasst.

8. Medikamentenabgabevorrichtung nach einem der Ansprüche 1-6, wobei die Kanüle (54, 614) einen harten oder weichen Katheter (120) umfasst.

9. Medikamentenabgabevorrichtung nach Anspruch 1, die ferner einen Nadeleinführungsmechanismus (56) umfasst, der funktionell mit der Kanüle (54, 614) gekoppelt ist, um die Kanüle zu bewegen zwischen einer eingezogenen Position, in der das Endstück (58, 618) der Kanüle im Lagerungszustand im Gehäuse (52, 610) verborgen ist, und einer ausgefahrenen Position, in der das Endstück der Kanüle im Abgabezustand aus dem Gehäuse heraus und über die distale Oberfläche (102, 625) hinausragt.

10. Medikamentenabgabevorrichtung nach Anspruch 1, wobei das Gehäuse (52, 610) einen einziehbaren Nadelschutz (622) umfasst, der die distale Oberfläche (102, 625) definiert, wobei der Nadelschutz im Aufbewahrungszustand eine ausgefahrene Position einnimmt, die das Endstück (58, 618) der Kanüle (54, 614) verdeckt, und im Abgabezustand eine eingefahrene Position einnimmt, die es dem Endstück der Kanüle ermöglicht, aus dem Gehäuse herauszuragen.

## Revendications

1. Dispositif d'administration de médicament, comprenant :
un boîtier (52, 610) définissant une cavité intérieure et une surface distale (102, 625) destinée à entrer en contact avec un patient durant une opération de dispensation du dispositif d'administration de médicament,
un récipient primaire (62, 612) disposé dans la cavité intérieure du boîtier et adapté pour contenir un médicament ;
une canule (54, 614) ayant une partie électroconductrice, la canule ayant un état de stockage où une extrémité terminale (58, 618) de celle-ci est à l'intérieur du boîtier et un état d'administration où l'extrémité terminale de celle-ci s'étend à l'extérieur à travers une ouverture (107, 607) dans la surface distale du boîtier ;
un chemin de débit de fluide (62, 663) pour le médicament incluant le récipient primaire et la canule ;
un mécanisme d'administration (150, 630) disposé dans le boîtier pour sélectivement forcer le médicament à travers le chemin de débit de fluide hors du récipient primaire et à travers la canule pour l'administration à un patient lorsque la canule occupe l'état d'administration ;
un dispositif de mesure de débit (61, 661) configuré pour mesurer une caractéristique du débit du médicament forcé à travers le chemin de débit de fluide ;
une électrode (103) disposée sur la surface distale du boîtier ; et
une unité de commande (60, 660) supportée par le boîtier et électriquement connectée au dispositif de mesure de débit, à la canule, et à l'électrode, l'unité de commande étant configurée pour déterminer si le médicament a été administré avec succès à un patient en :
déterminant qu'un circuit électrique (105, 605) incluant la canule et l'électrode s'est fermé à la suite de l'application de l'électrode sur la surface distale du boîtier sur la peau d'un patient et de l'insertion de la canule dans le patient ; et
déterminant, sur la base des mesures du dispositif de mesure de débit et en association avec la reconnaissance du circuit électrique fermé, si une dose complète du médicament a été forcée à travers le chemin de débit de fluide et administrée au patient.

2. Dispositif d'administration de médicament de la revendication 1, dans lequel le dispositif de mesure de débit (61, 661) comprend un capteur de débit fonctionnellement couplé au chemin de débit de fluide (63, 663) pour mesurer le débit du médicament à travers celui-ci.

3. Dispositif d'administration de médicament de la revendication 1 ou 2, dans lequel la partie électroconductrice de la canule (54, 614) est à une extrémité distale de celle-ci, et l'unité de commande (60, 660) est configurée pour déterminer une profondeur de l'extrémité distale de la canule dans l'état d'administration sur la base de résultats du circuit électrique (105, 625).

4. Dispositif d'administration de médicament de l'une quelconque des revendications précédentes, comprenant en outre un module de communication (183, 683), dans lequel l'unité de commande (60, 660) est configurée pour envoyer un signal de confirmation avec le module de communication en réponse au fait de déterminer que le circuit électrique (105, 625) était fermé alors qu'une dose complète du médicament était dispensée.

5. Dispositif d'administration de médicament de la revendication 4, dans lequel l'unité de commande (60, 660) est en outre configurée pour envoyer un signal de défaillance avec le module de communication (183, 683) en réponse au fait de déterminer que moins d'une dose complète du médicament a été dispensée ou que le circuit électrique (105, 625) s'est ouvert alors que le médicament était en train d'être dispensé.

6. Dispositif d'administration de médicament de l'une quelconque des revendications précédentes, dans lequel l'unité de commande (60, 660) est configurée pour :
commander le fonctionnement du mécanisme d'administration (150, 630) ; et
interrompre l'administration du médicament en réponse à au moins un de :
le fait de déterminer que le circuit électrique (105, 625) s'est ouvert avant qu'une dose complète du médicament a été dispensée ; ou
le fait de déterminer que la canule (54, 614) est à une profondeur incorrecte.

7. Dispositif d'administration de médicament de l'une quelconque des revendications précédentes, dans lequel la canule (54, 614) comprend une aiguille d'un matériau conducteur.

8. Dispositif d'administration de médicament de l'une quelconque des revendications 1 à 6, dans lequel la canule (54, 614) comprend un cathéter rigide ou flexible (120).

9. Dispositif d'administration de médicament de la revendication 1, comprenant en outre un mécanisme d'insertion d'aiguille (56) fonctionnellement accouplé à la canule (54, 614) pour déplacer la canule entre une position rétractée où l'extrémité terminale (58, 618) de la canule est dissimulée dans le boîtier (52, 610) dans l'état de stockage, et une position étendue où l'extrémité terminale de la canule s'étend à l'extérieur du boîtier et au-delà de la surface distale (102, 625) dans l'état d'administration.

10. Dispositif d'administration de médicament de la revendication 1, dans lequel le boîtier (52, 610) comprend un protège-aiguille rétractable (622) définissant la surface distale (102, 625), le protège-aiguille occupant une position rallongée dissimulant l'extrémité terminale (58, 618) de la canule (54, 614) dans l'état de stockage et une position rétractée permettant à l'extrémité terminale de la canule de s'étendre à l'extérieur du boîtier dans l'état d'administration.
